(19) Europäisches Patentamt
European Patent Office
Office européen des brevets

(11) EP 1 147 760 A2

(12) # EUROPÄISCHE PATENTANMELDUNG

(43) Veröffentlichungstag:
**24.10.2001 Patentblatt 2001/43**

(51) Int Cl.[7]: **A61K 7/00**, A61K 7/02

(21) Anmeldenummer: **01108565.1**

(22) Anmeldetag: **05.04.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **18.04.2000 DE 10019210**

(71) Anmelder: **Beiersdorf AG**
**20245 Hamburg (DE)**

(72) Erfinder:
- **Bleckmann, Andreas**
  **22926 Ahrensburg (DE)**
- **Kröpke, Rainer**
  **22869 Schenfefeld (DE)**
- **Syskowski, Boris**
  **22303 Hamburg (DE)**

(54) **Zubereitungen vom Emulsionstyp W/O mit niedrigviskosen gut spreitenden Lipidkomponenten**

(57) Wasser-in-Öl-Emulsionen

(a) mit einem Gehalt der Wasserphase von mindestens 80 Gew.-%, bezogen auf das Gesamtgewicht der Emulsionen,
(b) bei welchen die Lipidphase mindestens ein Lipid mit einer Viskosität von weniger als 15 mPa·s (bei 25 °C), welches einen Spreitwert von mindestens 700 $mm^2$/10 Minuten (bei 25 °C) aufweist, enthält,
(c) enthaltend wenigstens eine grenzflächenaktive Substanz, gewählt aus der Gruppe der Substanzen der der allgemeinen Formel (I)

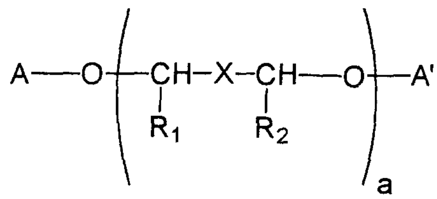

,

(d) gewünschtenfalls ferner enthaltend ein oder mehrerere kationische, nichtionische und/oder anionische Polymere, bevorzugt in Konzentrationen von 0,01 bis 10 Gew.%, vorzugsweise 0,1 bis 5 Gew.%, besonders bevorzugt 0,25 bis 1 Gew.%,

EP 1 147 760 A2

**Beschreibung**

Die vorliegende Erfindung betrifft kosmetische und dermatologische Zubereitungen, insbesondere solche vom Typ Wasser-in-Öl, Verfahren zu ihrer Herstellung sowie ihre Verwendung für kosmetische und medizinische Zwecke.

Die menschliche Haut übt als größtes Organ des Menschen zahlreiche lebenswichtige Funktionen aus. Mit durchschittlich etwa 2 $m^2$ Oberfläche beim Erwachsenen kommt ihr eine herausragende Rolle als Schutz- und Sinnesorgan zu. Aufgabe dieses Organs ist es, mechanische, thermische, aktinische, chemische und biologische Reize zu vermitteln und abzuwehren. Außerdem kommt ihr eine bedeutende Rolle als Regulations- und Zielorgan im menschlichen Stoffwechsel zu.

Unter kosmetischer Hautpflege ist in erster Linie zu verstehen, die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) zu stärken oder wiederherzustellen sowie ihre Hornschicht bei aufgetretenen Schäden in ihrem natürlichen Regenerationsvermögen zu unterstützen.

Werden die Barriereeigenschaften der Haut gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Natürlich ist dem Fachmann eine Vielzahl von Möglichkeiten bekannt, stabile W/O-Zubereitungen zur kosmetischen oder dermatologischen Anwendung zu formulieren, beispielsweise in Form von Cremes und Salben, die im Bereich von Raum- bis Haut-temperatur streichfähig sind, oder als Lotionen und Milche, die in diesem Temperaturbereich eher fließfähig sind. Der Stand der Technik kennt allerdings nur wenige Formulierungen, die so dünnflüssig sind, daß sie beispielsweise sprühbar wären.

Zudem haben dünnflüssige Zubereitungen des Standes der Technik häufig den Nachteil, daß sie instabil, auf einen engen Anwendungsbereich oder eine begrenzte Einsatzstoffauswahl begrenzt sind. Dünnflüssige Produkte, in denen beispielsweise stark polare Öle - wie die in handelsüblichen Produkten sonst häufig verwendeten Pflanzenöle - ausreichend stabilisiert sind, gibt es daher zur Zeit auf dem Markt nicht.

Unter dem Begriff "Viskosität" versteht man die Eigenschaft einer Flüssigkeit, der gegenseitigen laminaren Verschiebung zweier benachbarter Schichten einen Widerstand (Zähigkeit, innere Reibung) entgegenzusetzen. Man definiert heute diese sogenannte dynamische Viskosität nach $\eta=\tau/D$ als das Verhältnis der Schubspannung zum Geschwindigkeitsgradienten senkrecht zur Strömungsrichtung. Für newtonsche Flüssigkeiten ist $\eta$ bei gegebener Temperatur eine Stoffkonstante mit der SI-Einheit Pascalsekunde (Pa·s).

Der Quotient $\nu=\eta/\rho$ aus der dynamischen Viskosität $\eta$ und der Dichte $\rho$ der Flüssigkeit wird als kinematische Viskosität $\nu$ bezeichnet und in der SI-Einheit $m^2/s$ angegeben.

Als Fluidität ($\varphi$) bezeichnet man den Kehrwert der Viskosität ($\varphi=1/\eta$). Bei Salben und dergleichen wird der Gebrauchswert unter anderem mitbestimmt von der sogenannten Zügigkeit. Unter der Zügigkeit einer Salbe oder Salbengrundlage oder dergleichen versteht man deren Eigenschaft, beim Abstechen verschieden lange Fäden zu ziehen; dementsprechend unterscheidet man kurz- und langzügige Stoffe.

Während die graphische Darstellung des Fließverhaltens newtonscher Flüssigkeiten bei gegebener Temperatur eine Gerade ergibt, zeigen sich bei den sogenannten nicht-newtonschen Flüssigkeiten in Abhängigkeit vom jeweiligen Geschwindigkeitsgefälle D (Schergeschwindigkeit $\dot{\gamma}$) bzw. der Schubspannung $\tau$ oft erhebliche Abweichungen. In diesen Fällen läßt sich die sogenannte scheinbare Viskosität bestimmen, die zwar nicht der Newtonschen Gleichung gehorcht, aus der sich jedoch durch graphische Verfahren die wahren Viskositätswerte ermitteln lassen.

Die Fallkörperviskosimetrie ist lediglich zur Untersuchung newtonscher Flüssigkeiten sowie von Gasen geeignet. Sie basiert auf dem Stokes-Gesetz, nach dem für das Fallen einer Kugel durch eine sie umströmende Flüssigkeit die dynamische Viskosität $\eta$ aus

$$\eta = \frac{2r^2\,(\rho_K - \rho_{Fl})\cdot g}{9\cdot v}$$

bestimmbar ist, wobei

r = Radius der Kugel, v = Fallgeschwindigkeit, $\rho_K$ = Dichte der Kugel, $\rho_{Fl}$ = Dichte der Flüssigkeit und g = Fallbeschleunigung.

Die im Rahmen der vorliegenden Offenbarung aufgebführten Viskositätswerte der Zubereitungen und Einzelsubstanzen wurden mit Hilfe eines Viskosimeters des Typs Viskotester VT 02 der Gesellschaft Haake ermittelt.

W/O-Emulsionen mit hohem Wassergehalt und einer geringen Viskosität, die darüberhinaus eine Lagerstabilität aufweisen, wie sie für marktgängige Produkte gefordert wird, sind nach dem Stand der Technik nur sehr aufwendig zu formulieren. Dementsprechend ist das Angebot an derartigen Formulierungen äußerst gering. Gleichwohl könnten derartige Formulierungen dem Verbraucher bisher nicht gekannte kosmetische Leistungen bieten.

Eine Aufgabe der vorliegenden Erfindung war es, Zubereitungen zur Verfügung zu stellen, welche eine sehr geringe Viskosität haben und nicht die Nachteile des Standes der Technik aufweisen.

Eine weitere Aufgabe der vorliegenden Erfindung war es, Zubereitungen zur Verfügung zustellen, welche mit einem hohen Gehalt an wasserlöslichen und/oder wassermischbaren Substanzen mit kosmetischer oder dermatologischer Wirksamkeit beladen werden können, ohne daß die galenische Qualität oder andere Eigenschaften der Zubereitungen beeinträchtigt wären.

Überraschend hat sich gezeigt, daß Wasser-in-Öl-Emulsionen

(a) mit einem Gehalt der Wasserphase von mindestens 80 Gew.-%, bezogen auf das Gesamtgewicht der Emulsionen,
(b) bei welchen die Lipidphase mindestens ein Lipid mit einer Viskosität von weniger als 15 mPa·s (bei 25 °C), welches einen Spreitwert von mindestens 700 $mm^2$/10 Minuten (bei 25 °C) aufweist, enthält,
(c) enthaltend wenigstens eine grenzflächenaktive Substanz, gewählt aus der Gruppe der Substanzen der der allgemeinen Formel (I)

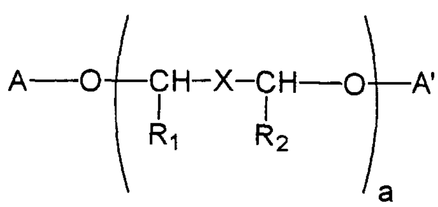

,

- wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema

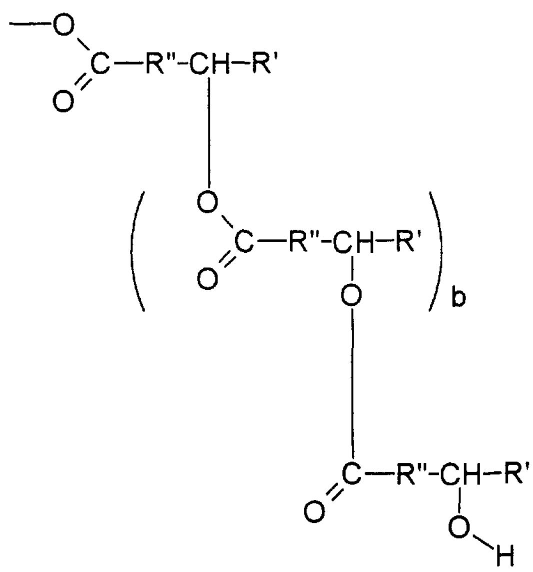

wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann,

- a eine Zahl von 1 bis 100, vorzugsweise 2 bis 60, insbesondere 5 bis 40 darstellt,
- X eine Einfachbindung oder die Gruppe

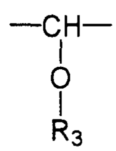

- darstellt,
- $R_1$ und $R_2$ unabhängig voneinander aus der Gruppe H, Methyl gewählt werden,
- $R_3$ gewählt wird aus der Gruppe H, sowie der verzweigten und unverzweigten, gesättigten und ungesättigten Alkyl- und Acylreste mit 1 - 20 Kohlenstoffatomen,

(d) gewünschtenfalls ferner enthaltend ein oder mehrerere kationische, nichtionische und/oder anionische Polymere, bevorzugt in Konzentrationen von 0,01 bis 10 Gew.%, vorzugsweise 0,1 bis 5 Gew.%, besonders bevorzugt 0,25 bis 1 Gew.%,

diese Aufgaben zu lösen vermögen.

Die Strukturformel ist nicht so zu interpretieren, daß durch den Index a alle in der Klammer repräsentierten Reste $R_1$, $R_2$ bzw $R_3$ im gesamten Molekül jeweils gleich sein müssen. Vielmehr können diese Reste in jedem der a Fragmente

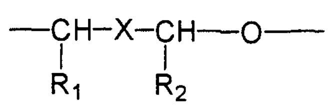

frei gewählt werden.

Es ist möglich und vorteilhaft, den Gesamtgehalt an Wasser und wasserlöslichen Substanzen der erfindungsgemäßen W/O-Emulsionen auf größer als 80 Gew.-%, insbesondere größer als 85 Gew.-% zu wählen, jeweils bezogen auf das Gesamtgewicht der Zubereitungen.

Ein Beispiel für besonders vorteilhaft im Sinne der vorliegenden Erfindung zu verwendende grenzflächenaktiven Substanzen ist das Polyethylenglycol-30-Dipolyhydroxystearat (PEG-30-Dipolyhydroxystearat), welches von der

Gesellschaft ICI Surfactants unter der Warenbezeichnung ARLACEL® P135 verkauft wird.

Die Gesamtmenge an den erfindungsgemäß verwendeten grenzflächenaktiven Substanzen in den fertigen kosmetischen oder dermatologischen Zubereitungen wird vorteilhaft aus dem Bereich von 0,1 - 30 Gew.-%, bevorzugt 0,25 - 5,0 Gew.-% insbesondere 0,75 - 3,5 Gew.-% gewählt, bezogen auf das Gesamtgewicht der Zubereitungen.

Zwar ist bekannt, daß sich mit Emulgatoren der vorab beschriebenen Art W/O-Emulsionen mit hohem Wassergehalt erzeugen lassen. Dennoch konnte der bekannte Stand der Technik nicht den Weg zur vorliegenden Erfindung weisen.

Es war für den Fachmann daher nicht vorauszusehen gewesen, daß die erfindungsgemäßen Zubereitungen

- besser als feuchtigkeitsspendende Zubereitungen wirken,
- einfacher zu formulieren sein,
- besser die Hautglättung fördern,
- sich durch besser Pflegewirkung auszeichen,
- besser als Vehikel für kosmetische und medizinisch-dermatologische Wirkstoffe dienen
- bessere sensorische Eigenschaften, wie beispielsweise die Verteilbarkeit auf der Haut oder das Einzugsvermögen in die Haut, aufweisen würden
- höhere Stabilität gegenüber Zerfall in Öl- und Wasserphasen aufweisen und
- sich durch bessere Bioverträglichkeit auszeichnen würden

als die Zubereitungen des Standes der Technik.

Es ist möglich und vorteilhaft, den Wassergehalt der erfindungsgemäßen W/O-Emulsionen auf deutlich größer als 80 Gew.-%, insbesondere größer als 85 Gew.-% zu wählen, jeweils bezogen auf das Gesamtgewicht der Zubereitungen.

Spreiten ist die oft erwünschte, in anderen Situationen auch oftmals unerwünschte vorwiegend auf Kapillarkräften beruhende Eigenschaft niedrigviskoser Öle, sich besonders leicht auf Unterlagen oder auch auf der Haut dünnschichtig zu verteilen. Dies kann bei der Hautpflege von Vorteil sein. Nachteilig kann sich diese Eigenschaft bei der Verpackung derartiger Öle oder diese enthaltender Zubereitungen auswirken. Ein Maß für das Spreitvermögen ist der Spreitungskoeffizient, der beispielsweise bei Entschäumern und Schaumverhütungsmitteln besonders hohe Werte annimmt.

Barry und Grace entwickelten eine Methode zur Bestimmung des Spreitverhaltens (J. Pharmac. Sci. 61, 335 [1972] und Beyer entwickelte ein Modell-Testsystem zur Prüfung des Spreitverhaltens (Arch. Pharm. [Weinh.] 310, 729 [1977]; Chem.Abstr. 88, Nr. 12-79017 [1978]). Über das Spreiten von Salben am Modell berichtet Beyer ferner in Arch. Pharm. 310, 473 und 858 (1977); Zbl. Pharm. 118, 51 (1979). Über das Spreitvermögen verschiedener flüssiger Hilfsstoffe auf Basis von Fetten bzw. fettähnlichen Stoffen berichten Pascale et.al. (Cosmet. Toiletries 100, Nr. 10, 75 [1985]).

Die Maßeinheit des Spreitungskoeffizienten ist die des Quotienten aus der Spreitungsfläche, über die die Spreitung erfolgt, und der Spreitungszeit, in der die Spreitung erfolgt. Sie wird üblicherweise in [$mm^2$/ 10 Minuten] angegeben.

Im Rahmen der vorliegenden Offenbarung wird als Oberbegriff für Fette, Öle, Wachse und dergleichen gelegentlich der Ausdruck "Lipide" verwendet, wie dem Fachmanne durchaus geläufig ist. Auch werden die Begriffe "Ölphase" und "Lipidphase" synonym angewandt.

Bevorzugt enthalten erfindungsgemäße Zubereitungen bis zu 35 Gew.-% einer Lipidphase.

Gemäß der hiermit vorgelegten Lehre sind vorteilhaft W/O-Emulsionen erhältlich, deren Viskosität bei 25 °C kleiner als 5.000 mPa·s (= Millipascalsekunden), insbesondere kleiner als 2.500 mPa·s, bevorzugt kleiner als 1.500 mPa·s ist (gemessen mit Viscotester VT-02, Haake).

Vorteilhaft werden die erfindungsgemäß verwendeten Öle gewählt aus der Gruppe der Substanzen, die in der folgenden Tabelle aufgelistet sind:

Tabelle 1

| Handelsname | Name analog INCI | Viskosität mPas | Spreitfähigkeit (20μl/Rotbandfilter) |
|---|---|---|---|
| | | mPa·s | $mm^2$/10 Min. |
| Solvent IH | Isohexadecan | 8 | 990 |
| | Isoeikosan | 12 | 800 |
| Cegesoft® C24 | Octylpalmitate | 11 | 910 |
| | Isopropylstearat | 9 | 910 |

Tabelle 1 (fortgesetzt)

| Handelsname | Name analog INCI | Viskosität mPas | Spreitfähigkeit (20µl/Rotbandfilter) |
|---|---|---|---|
| | | mPa·s | $mm^2$/10 Min. |
| Estol® 1540 EHC | Octylcocoat | 10 | 930 |
| Finsolv® TN | $C_{12-15}$ Alkylbenzoate | 14 | 730 |
| Cetiol® OE | Dicaprylylether | 8 | 1.020 |
| DUB DNPG | Neopentyl-Glycol-diheptanoat | 13 | 830 |
| Miglyol® 840 | Propylenglycol-dicaprylat/dicaprat | 12 | 855 |
| DC Fluid 345 | Cyclomethicon | 5 | 770 |
| | Isopropylpalmitat | 7,1 | 1.590 |
| Cetiol® B | Dibutyladipat | 5,5 | 935 |
| DUB VCI 10 | Isodecylneopentanoat | 3,9 | 962 |
| Cetiol® CC | Dicaprylylcarbonat | 7,26 | 875 |
| Cetiol® S | Dioctylcyclohexan | 14,41 | 723 |
| | Dihexylcarbonat | 3,8 | 1.056 |
| | Dihexylether | 1,87 | 1.174 |
| Ecolane® 130 | Cycloparaffin | 5,22 | 908 |
| Softcutol® O | Ethoxydiglycololeate | 13,07 | 804 |
| Transcutol® CG | Ethoxydiglycol | 4,14 | 999 |
| Dermofeel® BGC | Butylenglycol-caprylat/caprat | 12 | 800 |
| Prisorine® 2036 | Octylisostearat | 15 | 800 |
| Tegosoft® SH | Stearylheptanoat | 13 | 755 |
| Tegosoft® DC | Decylcocoat | 10,3 | 788 |
| Transcutol® P | Ethoxydiglycol | 4,27 | 962 |
| Arlasolv® DMI | Dimethylisosorbid | 10 | 880 |

Insbesondere bevorzugt sind Dicaprylylcarbonat, Dibutyladipat, Octylcocoat (= Ethylhexylcocoat), Octylpalmitat (= Ethylhexylpalmitat), Octylisostearat (= Ethylhexylisostearat).

Demgemäß ist erfindungsgemäß bevorzugt, einen Gehalt des Lipids oder der Lipide mit einer Viskosität von weniger als 15 mPa·s (bei 25 °C), welches einen Spreitwert von mindestens 700 $mm^2$/10 Minuten (bei 25 °C) aufweist, in der Lipidphase gemäß der Erfindung von mindestens etwa 50 Gew.-%, vorteilhaft mindestens etwa 75 Gew.-%, insbesondere vorteilhaft mindestens 80 Gew.-% zu wählen, jeweils bezogen auf die Gesamtlipidphase.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder-monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin.

Ein besonderer Vorzug der vorliegenden Erfindung ist es, daß sie gestattet, hohe Kohzenrationen an Polyolen, insbesondere Glycerin einzusetzen.

Bevorzugt enthalten Emulsionen gemäß der Erfindung ein oder mehrere Hydrokolloide.

"Hydrokolloid" ist die technologische Kurzbezeichnung für die an sich richtigere Bezeichnung "hydrophiles Kolloid". Hydrokolloide sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche

wasserlöslichen Polymere stellen eine große Gruppe chemisch sehr unterschiedlicher natürlicher und synthetischer Polymere dar, deren gemeinsames Merkmal ihre Löslichkeit in Wasser bzw. wäßrigen Medien ist. Voraussetzung dafür ist, daß diese Polymere über eine für die Wasserlöslichkeit ausreichende Anzahl an hydrophilen Gruppen besitzen und nicht zu stark vernetzt sind. Die hydrophilen Gruppen können nichtionischer, anionischer oder kationischer Natur sein, beispielsweise wie folgt:

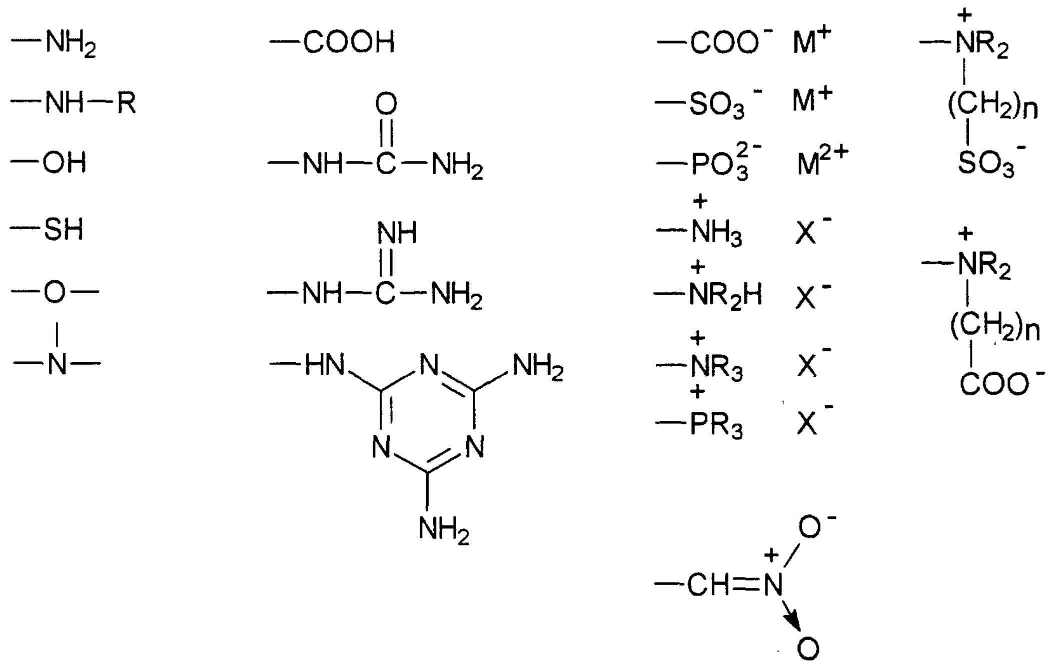

Die Gruppe der kosmetisch und dermatologisch relevanten Hydrokolloide läßt sich wie folgt einteilen in:

- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide,
- anorganische Verbindungen, wie z. B. Polykieselsäuren, Tonmineralien wie Montmorillonite, Zeolithe, Kieselsäuren.

Erfindungsgemäß bevorzugte Hydrokolloide sind beispielsweise Methylcellulosen, als welche die Methylether der Cellulose bezeichnet werden. Sie zeichnen sich durch die folgende Strukturformel aus

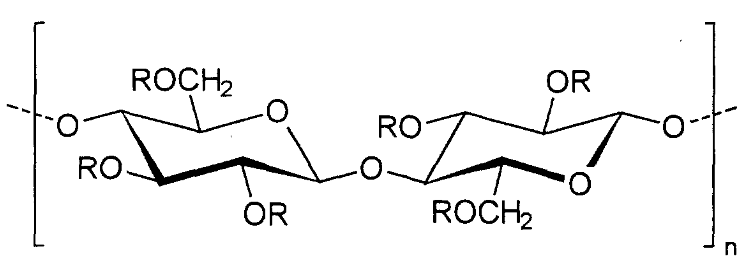

**Strukturformel I**

in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder $CH_2$-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von $2 \times 10^6$ bis $24 \times 10^6$ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Species unter aeroben Bedingungen mit einem Molekulargewicht von 2-15 $10^6$ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan wird anschließend getrocknet und gemahlen.

Vorteilhafter Gelbildner im Sinne der vorliegenden Erfindung ist ferner Carrageen, ein gelbildender und ähnlich wie Agar aufgebauter Extrakt aus nordatlantischen, zu den Florideen zählenden Rotalgen (Chondrus crispus und Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen, das in warmem Wasser sehr leicht lösl. ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt . Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute ι-Carrageenan ist sowohl wasserlöslich als auch gelbildend. Weitere Carrageen-Typen werden ebenfalls mit griechischen Buchstaben bezeichnet: α, β, γ, μ, ν, ξ, π, ω, χ. Auch die Art vorhandener Kationen ($K^+$, $NH_4^+$, $Na^+$, $Mg^{2+}$, $Ca^{2+}$) beeinflußt die Löslichkeit der Carrageene.

Die Verwendung von Chitosan in kosmetischen Zubereitungen ist per se bekannt. Chitosan stellt ein partiell deacyliertes Chitin dar. Dieses Biopolymer hat u.a. filmbildende Eigenschaften und zeichnet sich durch ein seidiges Hautgefühl aus. Von Nachteil ist jedoch seine starke Klebrigkeit auf der Haut, die insbesondere - vorübergehend - während der Anwendung auftritt. Entsprechende Zubereitungen können dann im Einzelfalle nicht vermarktungsfähig sein, da sie vom Verbraucher nicht akzeptiert bzw. negativ beurteilt werden. Chitosan wird bekanntermaßen beispielsweise in der Haarpflege eingesetzt. Es eignet sich, besser als das ihm zugrundeliegende Chitin, als Verdicker oder Stabilisator und verbessert die Adhäsion und Wasserresistenz von polymeren Filmen. Stellvertretend für eine Vielzahl von Fundstellen des Standes der Technik: H.P.Fiedler, "Lexikon der Hilfsstoffe für Pharmazie, Kosmetik und angrenzende Gebiete", dritte Auflage 1989, Editio Cantor, Aulendorf, S. 293, Stichwort "Chitosan".

Chitosan ist gekennzeichnet durch folgende Strukturformel:

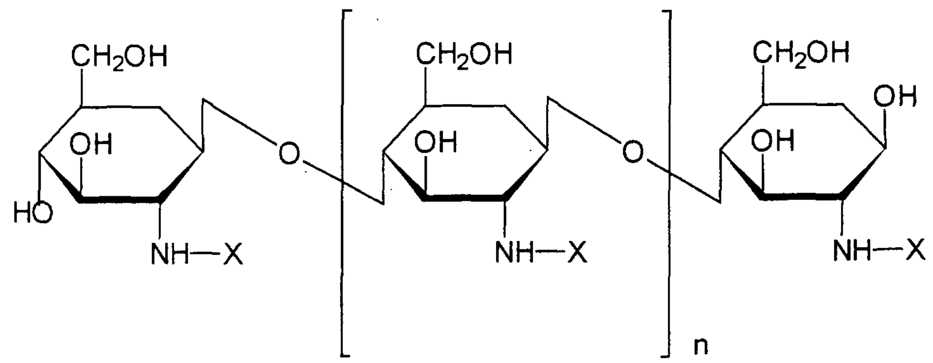

dabei nimmt n Werte bis zu ca. 10.000 an, X stellt entweder den Acetylrest oder Wasserstoff dar. Chitosan entsteht durch Deacetylierung und teilweise Depolymerisation (Hydrolyse) von Chitin, welches durch die Strukturformel

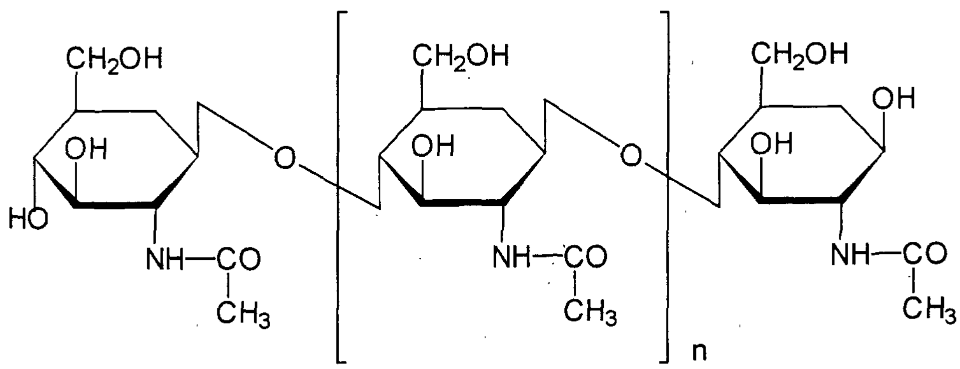

gekennzeichnet ist. Chitin ist wesentlicher Bestandteil des Ektoskeletts ['o χιω = grch.: der Panzerrock] der Gliederfüßer (z.B. Insekten, Krebse, Spinnen) und wird auch in Stützgeweben anderer Organismen (z.B. Weichtiere, Algen, Pilze) gefunden.

Im Bereich von etwa pH <6 ist Chitosan positiv geladen und dort auch in wäßrigen Systemen löslich. Es ist nicht kompatibel mit anionischen Rohstoffen. Daher bietet sich zur Herstellung chitosanhaltiger Öl-in-Wasser-Emulsionen der Einsatz nichtionischer Emulgatoren an. Diese sind an sich bekannt, beispielsweise aus der EP-A 776 657.

Erfindungsgemäß bevorzugt sind Chitosane mit einem Deacetylierungsgrad > 25 %, insbesondere > 55 bis 99 % [bestimmt mitteis $^{1}$H-NMR]).

Es ist von Vorteil, Chitosane mit Molekulargewichten zwischen 10.000 und 1.000.000 zu wählen, insbesondere solches mit Molekulargewichten zwischen 100.000 und 1.000.000. [bestimmt mittels Gelpermetionschromatographie].

Polyacrylate sind ebenfalls vorteilhaft im sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

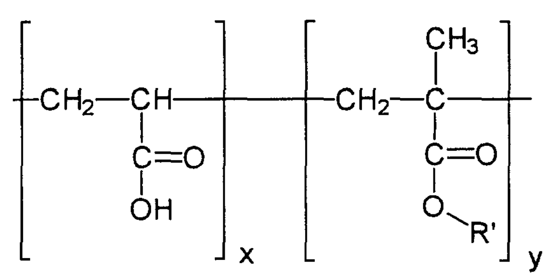

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F.Goodrich Company erhältlich sind.

Ferner vorteilhaft sind Copolymere aus $C_{10-30}$-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/C 10-30 Alkyl Acrylate Crosspolymer" tragen. Insbesondere vorteilhaft sind die unter den Handelsbezeichnungen Pemulen TR1 und Pemulen TR2 bei der B. F. Goodrich Company erhältlichen.

Die Gesamtmenge an einem oder mehreren Hydrokolloiden wird in den fertigen kosmetischen oder dermatologischen Zubereitungen vorteilhaft kleiner als 1,5 Gew.-%, bevorzugt zwischen 0,1 und 1,0 Gew.-%, bezogen auf das Gesamtgewicht der Zubereitungen, gewählt.

Hydrokolloide aus der Gruppe der anionischen Polymere werden vorteilhaft im Sinne der vorliegenden Erfindung gewählt aus der Gruppe der Carbomere als Natrium-, Kalium-, TEA- und Trisamino-Salze, Natrium-, Kaliumhyaluronat, Microcrystalline Cellulose + Cellulose Gum, Veegum-Typen, Hektorite, Bentonite, Laponite, Alginate, Methacrylate.

Hydrokolloide aus der Gruppe der nichtionischen Polymere werden vorteilhaft im Sinne der vorliegenden Erfindung gewählt aus der Gruppe Polyvinylpyrolidon, Hydroxypropyl Methylcellulose, Polyvinylalkohol, Polyether-1, Xanthan Gum, Hydroxyethylcellulose, Cellulosederivate, Stärke, Stärkederivate, Guar Gum, Glycerylmethacrylat.

Hydrokolloide aus der Gruppe der kationischen Polymere werden vorteilhaft im Sinne der vorliegenden Erfindung gewählt aus der Gruppe Chitosan, kationische Stärkederivate, kationische Cellulosederivate, Guar-Hydroxypropyltrimethylammoniumchlorid, Natriumpolystyrolsulfonat

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin, $\Psi$-Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$ mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmann natürlich bekannt, daß kosmetische Zubereitungen zumeist nicht ohne die üblichen Hilfs- und Zusatzstoffe denkbar sind. Die erfindungsgemäßen kosmetischen und dermatologischen Zubereitungen können dementsprechend ferner kosmetische Hilfsstoffe enthalten, wie sie üblicherweise in solchen Zubereitungen verwendet werden, beispielsweise Konsistenzgeber, Stabilisatoren, Füllstoffe, Konservierungsmittel, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Wasser, Salze, antimikrobiell, proteolytisch oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte.

Letztere können beispielsweise gewählt werden aus der Gruppe der Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren, Ethylendiamintetraessigsäure und deren Salze und andere mehr. Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus.

Mutatis mutandis gelten entsprechende Anforderungen an die Formulierung medizinischer Zubereitungen.

Die erfindungsgemäßen W/O-Emulsionen können als Grundlage für kosmetische oder dermatologische Formulierungen dienen. Diese können wie üblich zusammengesetzt sein und beispielsweise zur Behandlung und der Pflege der Haut und/oder der Haare, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika oder Dermatika üblichen Weise auf die Haut und/oder die Haare in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die dünnflüssigen kosmetischen oder dermatologischen Mittel gemäß der Erfindung können beispielsweise als aus Aerosolbehältern, Quetschflaschen oder durch eine Pumpvorrichtung versprühbare Präparate vorliegen oder in Form einer mittels Roll-on-Vorrichtungen auftragbaren flüssigen Zusammensetzung, jedoch auch in Form einer aus normalen Flaschen und Behältern auftragbaren Emulsion.

Als Treibmittel für aus Aerosolbehältern versprühbare kosmetische oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UV-A-Filtersubstanz und/oder mindestens eine UV-B-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Metho-xyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- Derivate des 1,3,5-Triazins, vorzugsweise 2,4,6-Trianilino-(p-carbo-2'-ethyl-1'-hexyloxy) -1,3,5-triazin.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, erfindungsgemäße Lipodispersionen mit UVA-Filtem zu formulieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Erfindungsgemäße kosmetische und dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Als weitere Bestandteile können verwendet werden:

- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder-monobutylether, Diethylenglykolmonomethyl- oder -monoethylether und analoge Produkte.

Erfindungsgemäße Zubereitungen können auch Wirkstoffe (eine oder mehrere Verbindungen) enthalten, welche gewählt werden aus der Gruppe: Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin $B_1$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die γ-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter. Vorteilhaft ist es auch, die Wirkstoffe aus der Gruppe der rückfettenden Substanzen zu wählen, beispielsweise Purcellinöl, Eucerit® und Neocerit®.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Beispiel 1:**

| | Gew.-% |
|---|---|
| PEG-30 Dipolyhydroxystearat | 1,5 |
| Isohexadekan | 12.0 |
| Isoeicosan | 5,0 |
| Glycerin | 3,0 |
| NaCl | 1,0 |
| Parfüm, Konservierungsmittel, Farbstoffe, Antioxidantien usw. | q.s. |
| Wasser | ad 100,0 |

**Beispiel 2:**

| | Gew.-% |
|---|---|
| PEG-30 Dipolyhydroxystearat | 1,0 |
| Dicaprylylether | 4,5 |
| Octylcocoat | 6,5 |
| Glycerin | 3,0 |
| Magnesiumsulfat | 0,7 |
| Parfüm, Konservierungsmittel, Farbstoffe, Antioxidantien usw. | q.s. |
| Wasser | ad 100,0 |

**Beispiel 3 (W/O-Lotion):**

| | Gew.-% |
|---|---|
| PEG-30 Dipolyhydroxystearat | 2,0 |
| Isopropylstearat | 4,0 |
| $C_{12-15}$ Alkylbenzoate | 4,0 |
| Octylpalmitat | 4,0 |
| Glycerin | 3,0 |
| Xanthan Gum | 0.5 |
| NaCl | 1,0 |
| Parfüm, Konservierungsmittel, Farbstoffe, Antioxidantien usw. | q.s. |
| Wasser | ad 100,0 |

**Beispiel 4 :**

| | Gew.-% |
|---|---|
| PEG-30 Dipolyhydroxystearat | 2,0 |
| Neopentyl-Glycoldiheptanoat | 9,0 |
| Tocopherolacetat | 0,5 |
| Glycerin | 3,0 |
| Panthenol | 0,3 |
| 1,3 Butylenglycol | 1,0 |

(fortgesetzt)

| | Gew.-% |
|---|---|
| Serin | 0,3 |
| Biotin | 0,1 |
| Distärkephosphat | 1,0 |
| Magnesiumsulfat | 0,7 |
| Parfüm, Konservierungsmittel, Farbstoffe, Antioxidantien usw. | q.s. |
| Wasser | d 100,0 |

**Beispiel 5 :**

| | Gew.-% |
|---|---|
| PEG-30 Dipolyhydroxystearat | 2,0 |
| Dicaprylylether | 10,0 |
| Dibutyladipat | 5,0 |
| Butylmethoxydibenzoylmethan | 1,0 |
| Octylmethoxycinnamat | 2,0 |
| Methylbenzylidencampher | 2,0 |
| Octyltriazon | 0,5 |
| Titandioxid | 1,0 |
| Zinkoxid | 1,0 |
| Glycerin | 1,0 |
| Magnesiumsulfat | 0,7 |
| Parfüm, Konservierungsmittel, Farbstoffe, Antioxidantien usw. | q.s. |
| Wasser | ad 100,0 |

**Beispiel 6:**

| | Gew.-% |
|---|---|
| PEG-30 Dipolyhydroxystearat | 2,0 |
| Dicaprylylether | 4,5 |
| Isopropylpalmitat | 4,5 |
| Glycerin | 3,0 |
| Natriumchlorid | 0,7 |
| PVP | 2,0 |
| Parfüm, Konservierungsmittel, Farbstoffe, Antioxidantien usw. | q.s. |
| Wasser | ad 100,0 |

**Beispiel 7:**

| | Gew.-% |
|---|---|
| PEG-30 Dipolyhydroxystearat | 2,0 |
| Cycloparaffin | 4,0 |
| Ethoxydiglycololeat | 2,5 |
| Dioctylcyclohexan | 6.5 |

(fortgesetzt)

| | Gew.-% |
|---|---|
| Glycerin | 20,0 |
| Propylenglycol | 15,0 |
| Magnesiumsulfat | 0,7 |
| Parfüm, Konservierungsmittel, Farbstoffe, Antioxidantien usw. | q.s. |
| Wasser | ad 100,0 |

**Beispiel 8 (Emulsions-Make-up):**

| | Gew.-% |
|---|---|
| PEG-30 Dipolyhydroxystearat | 2,0 |
| Dicaprylylcarbonat | 2,0 |
| $C_{12-15}$ Alkylbenzoate | 9,0 |
| Squalan | 1,0 |
| Distärkephosphat | 0,5 |
| Cyclomethicon | 0,5 |
| Glycerin | 1,5 |
| Magnesiumsilikat | 1,0 |
| Natriumchlorid | 0,7 |
| Glimmer | 0,5 |
| Eisenoxide | 0,5 |
| Titandioxid | 1,0 |
| Talkum | 1,0 |
| Tapiokastärke | 0,25 |
| Parfüm, Konservierungsmittel, NaOH, Farbstoffe, Antioxidantien usw. | q.s. |
| Wasser | ad 100,0 |

**Beispiel 9 :**

| | Gew.-% |
|---|---|
| PEG-30 Dipolyhydroxystearat | 1,5 |
| Dicaprylylether | 10,0 |
| Glycerin | 3,0 |
| Magnesiumsulfat | 0,7 |
| Polyether-1 | 1,0 |
| Parfüm, Konservierungsmittel, Farbstoffe, Antioxidantien usw. | q.s. |
| Wasser | ad 100,0 |

**Beispiel 10 (W/O-Lotion):**

| | Gew.-% |
|---|---|
| PEG-30 Dipolyhydroxystearat | 1,5 |
| Dicaprylylcarbonat | 10,0 |
| Glycerin | 3,0 |

(fortgesetzt)

| | Gew.-% |
|---|---|
| Magnesiumsulfat | 0,7 |
| Parfüm, Konservierungsmittel, Farbstoffe, Antioxidantien usw. | q.s. |
| Wasser | ad 100,0 |

**Beispiel 11 (W/O-Lotion):**

| | Gew.-% |
|---|---|
| PEG-30 Dipolyhydroxystearat | 1,5 |
| Butylenglycol Caprylat/Caprat | 10,0 |
| Glycerin | 3,0 |
| Magnesiumsulfat | 0,7 |
| Parfüm, Konservierungsmittel, Farbstoffe, Antioxidantien usw. | q.s. |
| Wasser | ad 100,0 |

**Beispiel 12 (W/O-Lotion):**

| | Gew.-% |
|---|---|
| PEG-30 Dipolyhydroxystearat | 1,5 |
| Decylcocoat | 5,0 |
| Glycerin | 3,0 |
| Magnesiumsulfat | 0,7 |
| Parfüm, Konservierungsmittel, Farbstoffe, Antioxidantien usw. | q.s. |
| Wasser | ad 100,0 |

**Beispiel 13 (W/O-Lotion):**

| | Gew.-% |
|---|---|
| PEG-30 Dipolyhydroxystearat | 1,5 |
| Octylpalmitat | 10,0 |
| Octylstearat | 2,0 |
| Glycerin | 3,0 |
| Magnesiumsulfat | 0,7 |
| Parfüm, Konservierungsmittel, Farbstoffe, Antioxidantien usw. | q.s. |
| Wasser | ad 100,0 |

**Beispiel 14:**

| | Gew.-% |
|---|---|
| PEG-30 Dipolyhydroxystearat | 1,5 |
| Isopropylstearat | 10,0 |
| Glycerin | 3,0 |

(fortgesetzt)

| | Gew.-% |
|---|---|
| Magnesiumsulfat | 0,7 |
| Parfüm, Konservierungsmittel, Farbstoffe, Antioxidantien usw. | q.s. |
| Wasser | ad 100,0 |

**Beispiel 15 :**

| | Gew.-% |
|---|---|
| PEG-30 Dipolyhydroxystearat | 1,5 |
| Stearylheptanoat | 10,0 |
| Glycerin | 3,0 |
| Magnesiumsulfat | 0,7 |
| Parfüm, Konservierungsmittel, Farbstoffe, Antioxidantien usw. | q.s. |
| Wasser | ad 100,0 |

**Beispiel 16 :**

| | Gew.-% |
|---|---|
| PEG-30 Dipolyhydroxystearat | 1,5 |
| $C_{12-15}$ Alkylbenzoat | 10,0 |
| Glycerin | 3,0 |
| Magnesiumsulfat | 0,7 |
| Parfüm, Konservierungsmittel, Farbstoffe, Antioxidantien usw. | q.s. |
| Wasser | ad 100,0 |

**Beispiel 17 :**

| | Gew.-% |
|---|---|
| PEG-30 Dipolyhydroxystearat | 1,5 |
| Dimethylisosorbid | 10,0 |
| Glycerin | 3,0 |
| Magnesiumsulfat | 0,7 |
| Parfüm, Konservierungsmittel, Farbstoffe, Antioxidantien usw. | q.s. |
| Wasser | ad 100,0 |

**Beispiel 18 :**

| | Gew.-% |
|---|---|
| PEG-30 Dipolyhydroxystearat | 1,5 |
| Butylenglycol Caprylat/Caprat | 10,0 |
| Glycerin | 3,0 |
| Magnesiumsulfat | 0,7 |

(fortgesetzt)

| | Gew.-% |
|---|---|
| Parfüm, Konservierungsmittel, Farbstoffe, Antioxidantien usw. | q.s. |
| Wasser | ad 100,0 |

**Beispiel 19 :**

| | Gew.-% |
|---|---|
| PEG-30 Dipolyhydroxystearat | 1,5 |
| Dihexylether | 10,0 |
| Glycerin | 3,0 |
| Magnesiumsulfat | 0,7 |
| Parfüm, Konservierungsmittel, Farbstoffe, Antioxidantien usw. | q.s. |
| Wasser | ad 100,0 |

**Beispiel 20:**

| | Gew.-% |
|---|---|
| PEG-30 Dipolyhydroxystearat | 1,5 |
| Isodecylneopentanoat | 10,0 |
| Glycerin | 3,0 |
| Magnesiumsulfat | 0,7 |
| Parfüm, Konservierungsmittel, Farbstoffe, Antioxidantien usw. | q.s. |
| Wasser | ad 100,0 |

**Beispiel 21 :**

| | Gew.-% |
|---|---|
| PEG-30 Dipolyhydroxystearat | 1,5 |
| Propylenglycol Dicaprylat/Dicaprat | 10,0 |
| Glycerin | 3,0 |
| Magnesiumsulfat | 0,7 |
| Parfüm, Konservierungsmittel, Farbstoffe, Antioxidantien usw. | q.s. |
| Wasser | ad 100,0 |

**Beispiel 22 :**

| | Gew.-% |
|---|---|
| PEG-30 Dipolyhydroxystearat | 1,5 |
| Caprylic/Capric Diglyceryl Succinate | 10,0 |
| Glycerin | 3,0 |
| Magnesiumsulfat | 0,7 |
| Parfüm, Konservierungsmittel, Farbstoffe, Antioxidantien usw. | q.s. |

(fortgesetzt)

| | Gew.-% |
|---|---|
| Wasser | ad 100,0 |

**Beispiel 23 (W/O-Lotion):**

| | Gew.-% |
|---|---|
| PEG-30 Dipolyhydroxystearat | 1,5 |
| Octylisostearat | 10,0 |
| Glycerin | 3,0 |
| Magnesiumsulfat | 0,7 |
| Parfüm, Konservierungsmittel, Farbstoffe, Antioxidantien usw. | q.s. |
| Wasser | ad 100,0 |

**Beispiel 24 (W/O-Lotion):**

| | Gew.-% |
|---|---|
| PEG-30 Dipolyhydroxystearate | 2,0 |
| Isopropylstearat | 4,0 |
| $C_{12-15}$ Alkylbenzoate | 4,0 |
| Octylpalmitat | 4,0 |
| Glycerin | 3,0 |
| Guar-Hydroxypropyltrimethylammoniumchlorid | 0.5 |
| NaCl | 1,0 |
| Parfüm, Konservierungsmittel, Farbstoffe, Antioxidantien etc. | q.s. |
| Wasser | ad 100,0 |

**Beispiel 25:**

| | Gew.-% |
|---|---|
| PEG-30 Dipolyhydroxystearate | 2,0 |
| Dicaprylylether | 4,5 |
| Isopropylpalmitat | 4,5 |
| Glycerin | 3,0 |
| Natriumchlorid | 0,7 |
| Chitosan | 1,0 |
| Milchsäure | 0,6 |
| Parfüm, Konservierungsmittel, Farbstoffe, Antioxidantien etc. | q.s. |
| Wasser | ad 100,0 |

**Beispiel 26 :**

| | Gew.-% |
|---|---|
| PEG-30 Dipolyhydroxystearate | 1,5 |
| Dicaprylylether | 10,0 |
| Glycerin | 3,0 |
| Magnesiumsulfat | 0,7 |
| Natriumpolystyrolsulfonat | 1,0 |
| Parfüm, Konservierungsmittel, Farbstoffe, Antioxidantien etc. | q.s. |
| Wasser | ad 100,0 |

**Beispiel 27 (W/O-Lotion):**

| | Gew.-% |
|---|---|
| PEG-30 Dipolyhydroxystearate | 2,0 |
| Isopropylstearat | 4,0 |
| $C_{12-15}$ Alkyl Benzoate | 4,0 |
| Octylpalmitat | 4,0 |
| Glycerin | 3,0 |
| Hyaluronsäure, Na-Salz | 0.5 |
| NaCl | 1,0 |
| Parfüm, Konservierungsmittel, Farbstoffe, Antioxidantien etc. | q.s. |
| Wasser | ad 100,0 |

**Beispiel 28:**

| | Gew.-% |
|---|---|
| PEG-30 Dipolyhydroxystearate | 2,0 |
| Dicaprylylether | 4,5 |
| Isopropylpalmitat | 4,5 |
| Glycerin | 3,0 |
| Natriumchlorid | 0,7 |
| Carbomer | 0,6 |
| Parfüm, Konservierungsmittel, Farbstoffe, Antioxidantien etc. | q.s. |
| Wasser | ad 100,0 |

**Beispiel 29 :**

| | Gew.-% |
|---|---|
| PEG-30 Dipolyhydroxystearate | 1,5 |
| Dicaprylylether | 10,0 |
| Glycerin | 3,0 |
| Magnesiumsulfat | 0,7 |
| Carbomer | 1,0 |

(fortgesetzt)

| | Gew.-% |
|---|---|
| Natriumhydroxid 10% | 0.5 |
| Parfüm, Konservierungsmittel, Farbstoffe, Antioxidantien etc. | q.s. |
| Wasser | ad 100,0 |

## Patentansprüche

**1.** Wasser-in-Öl-Emulsionen

(a) mit einem Gehalt der Wasserphase von mindestens 80 Gew.-%, bezogen auf das Gesamtgewicht der Emulsionen,
(b) bei welchen die Lipidphase mindestens ein Lipid mit einer Viskosität von weniger als 15 mPa·s (bei 25 °C), welches einen Spreitwert von mindestens 700 $mm^2$/10 Minuten (bei 25 °C) aufweist, enthält,
(c) enthaltend wenigstens eine grenzflächenaktive Substanz, gewählt aus der Gruppe der Substanzen der der allgemeinen Formel (I)

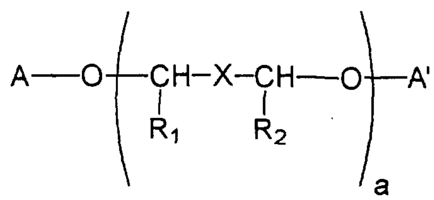

- wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema

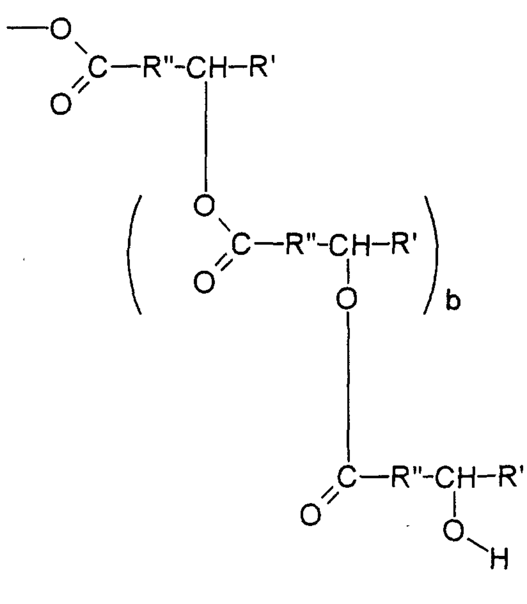

wobei R' gewählt wird aus der Gruppe der verzweigten und unverzweigten Alkylgruppen mit 1 bis 20 Kohlenstoffatomen und R'' gewählt wird aus der Gruppe der verzweigten und unverzweigten Alkylengruppen mit 1 bis 20 Kohlenstoffatomen und b Zahlen von 0 bis 200 annehmen kann,
- a eine Zahl von 1 bis 100, vorzugsweise 2 bis 60, insbesondere 5 bis 40 darstellt,
- X eine Einfachbindung oder die Gruppe

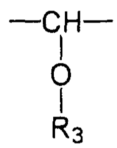

- darstellt,
- $R_1$ und $R_2$ unabhängig voneinander aus der Gruppe H, Methyl gewählt werden,
- $R_3$ gewählt wird aus der Gruppe H, sowie der verzweigten und unverzweigten, gesättigten und ungesättigten Alkyl- und Acylreste mit 1 - 20 Kohlenstoffatomen,

(d) gewünschtenfalls ferner enthaltend ein oder mehrerere kationische, nichtionische und/oder anionische Polymere, bevorzugt in Konzentrationen von 0,01 bis 10 Gew.%, vorzugsweise 0,1 bis 5 Gew.%, besonders bevorzugt 0,25 bis 1 Gew.%,

**2.** Emulsionen nach Anspruch 1, **dadurch gekennzeichnet, daß** der Gehalt der Wasserphase größer ist als 85 Gew.-%, jeweils bezogen auf das Gesamtgewicht der Zubereitungen.

**3.** Emulsionen nach Anspruch 1, **dadurch gekennzeichnet, daß** die grenzflächenaktive Substanz oder Substanzen aus der Gruppe Polyethylenglycol-30-Dipolyhydroxystearat und Polyglyceryl-2-Polyhydroxystearat gewählt wird oder werden.

**4.** Emulsionen nach Anspruch 1, **dadurch gekennzeichnet, daß** die Ölphase zu mindestens 50 Gew.-%, bevorzugt zu mehr als 80 Gew.-% aus mindestens einer Substanz, gewählt aus der Gruppe gewählt aus der Gruppe Isohexadecan, Isoeikosan, Octylpalmitate, Isopropylstearat, Octylcocoat, $C_{12-15}$-Alkylbenzoate, Dicaprylylether, Neopentyl-Glycol-diheptanoat, Propylenglycol-dicaprylat/dicaprat, Cyclomethicon, Isopropylpalmitat, Dibutyladipat, Isodecylneopentanoat, Dicaprylylcarbonat, Dioctylcyclohexan, Dihexylcarbonat, Dihexylether, Cycloparaffin, Ethoxydiglycololeate, Ethoxydiglycol, Butylenglycolcaprylat/caprat, Octylisostearat, Stearylheptanoat, Decylcocoat, Dimethylisosorbid besteht.